# EUROPEAN PATENT SPECIFICATION

(11) **EP 3 947 529 B1**
(45) Date of publication and mention of the grant of the patent: **06.08.2025**
(21) Application number: 20715390.9
(22) Date of filing: 03.04.2020
(51) Int. Cl.: C08H 1/06, C08L 89/06, B29C 64/00, C09D 11/04, C07K 14/78, B33Y 70/00, C09D 11/30, C09D 11/104

(54) **A GELATIN AND USES THEREOF**
GELATINE UND VERWENDUNGEN DAVON
GÉLATINE ET SES UTILISATIONS

(30) Priority: 03.04.2019 EP 19167094
(43) Date of publication of application: 09.02.2022
(73) Proprietor: Tessenderlo Group NV, 1050 Brussel (BE)
(72) Inventor: CONDI DE GODOI, Fernanda, 3001 Heverlee (BE); DIERCKX, Stephan, R., M., 1910 Berg (BE); QUANTEN, Erwin, Theo, Maria, 3020 Herent (BE); VANDENDRIESSCHE, Jan, Maurits, August, 1050 Brussel (BE)
(74) Representative: Hoyng Rokh Monegier B.V.
(86) International application number: PCT/EP2020/059669
(87) International publication number: WO 2020/201555

(56) References cited:
- EP-A1- 1 961 411
- US-A1- 2015 084 232
- US-B2- 9 101 681

## Description

### TECHNICAL FIELD

The present invention relates to a gelatin having improved properties, in particular improved dispensing properties, more particularly improved printing properties. The present invention further relates to a construct produced with the gelatin of the present invention and to a process to produce said construct. Further, the present invention relates to the use of the gelatin of the present invention to solve existing problems encountered with dispensing systems, in particular with 3D printing and more particularly for applications in the medical field. The gelatin of the present invention is particularly suitable for bio-printing in the medical field and may also be used in food applications.

### Background

Manufacturing technologies in the medical field and also in the food industry, are in constant evolution. Dispensing techniques, in particular, offer some advantages compared to standard production technologies, particularly in the medical field. Said advantages can be the speed of manufacturing, customization and the like. One very well-known dispensing technique is 3D printing. In particular, in the field of medical research, bioprinting is a powerful tool used for tissue engineering applications due to its capability of directly dispensing biological materials into spatial orientations and geometries. Gelatin is a preferred substrate biomaterial because of its very high biocompatibility with living tissues. Indeed, gelatin shows a natural amino acid sequence of Arginine-Glycine-Aspartate (Arg-Gly-Asp) component which is the main amino acid sequence responsible for cell adhesion in extracellular matrices (ECMs). In biology, an extracellular matrix (ECM) is a three-dimensional network of extracellular macromolecules, such as collagen, enzymes, and glycoproteins, that provide structural and biochemical support to surrounding cells.

An important parameter for dispensing systems is the dispensing time window, i.e. the time during which a material can be dispensed without showing detrimental viscosity increase upon dispensing, in particular, inside the dispensing head or nozzle. It is advantageous to have a longer dispensing time window as this gives more flexibility during the dispensing process. However, an important issue nowadays in dispensing technologies and particular in 3D printing the dispensing time window of materials based on gelatin is short because of the lack of thermostability. Gelatin-based formulations rapidly show an increase in viscosity after the start of the dispensing process, and this increase in viscosity causes the material to block the dispensing head. Dispensing can then only be done for a limited period of time before the dispensing head is blocked and has to be cleaned or changed before dispensing can be continued. This is not convenient at all to make bigger constructs or to make several smaller constructs. Thus with existing gelatin-based dispensing material, the dispensing time window is limited. In particular, gelatin, which as mentioned above is very suitable because of its biocompatibility with living tissues, is known to be a high-risk material for nozzle clogging. Due to the thermal instability of gelatin, a gelatin solution (prepared for printing typically at concentrations above 7.5%) will rapidly show an increase in viscosity at room temperature (20 to 25 °C), and will block dispensing heads, in particular those having a diameter below 0.64 mm (or 22G). Various solutions exist to overcome this problem. One solution is to dispense the material in a temperature-controlled dispensing system. However, such temperature controlled systems are expensive and require great care during operation, which makes the dispensing operation quite complicated. Another solution is to chemically modify the dispensing material or to supplement it with additives. Such currently available bio-printing materials include modified gelatins. Also gelatin can be mixed with other hydrocolloids such as alginates for example. Further, use of only specific raw materials such as fish gelatin may be a solution.

WO 2017216780 A1 relates to a gelatin polymer which is derived from natural sources of cold-adapted marine species, such as salmon gelatin.

US 9 101 681 B2 relates to the use of standard gelatin to make 3D constructs in a temperature-controlled dispensing system.

US 2015/0084232 A1 relates to hydrogel compositions for printing 3D objects, wherein the hydrogel comprises a cross-linked biocompatible polymer. Pre-crosslinking is done before extrusion to ensure stability of the printing material.

There is thus a need to have a dispensing material which is easy to use, e.g. which does not require temperature-controlled systems, which has a longer dispensing time window than existing materials, which may be dispensed at higher concentrations than what is currently possible and so on.

The present invention aims to solve at least the above mentioned problems.

### SUMMARY OF THE INVENTION

The present invention is based on the findings of a novel gelatin which has improved properties in particular in the field of dispensing systems (or devices), such as 3D printing systems (or devices), in particular for the medical field, in cosmetic applications and food applications, more particularly for the medical field. The gelatin of the present invention allows to have a longer dispensing time window, preferably printing time window, in dispensing systems, such as 3D printing systems. The gelatin of the present invention is thermally stable which is advantageous as it can be dispensed preferably at typical dispensing, or printing concentrations of about 7.5 to 15%, without the need of having complicated temperature controlling systems in place or the need of having complicated formulations with additives or bulking material.

Thus in a first aspect, the present invention relates to a gelatin characterized in that
a. It is soluble in an aqueous medium and
b. the first derivative of the FTIR-ATR spectrum remains below zero within the 1440-1355 cm⁻¹ spectral region.

In a second aspect, the present invention relates to the gelatin of the first aspect of the present invention for use in dispensing devices, preferably 3D printing devices.

In a third aspect, the present invention relates to a gelatin composition comprising the gelatin of the present invention and one or more further ingredients, from the group comprising bulking agents, hydrocolloids and proteins.

In a fourth aspect, the present invention relates to the gelatin composition of the third aspect of the present invention for use in dispensing devices, preferably 3D printing devices.

In a fifth aspect, the present invention relates to an aqueous gelatin composition comprising from 1 to 30wt% of the gelatin of the present invention or the gelatin composition of the present invention and from 70 to 99wt% of water.

In a sixth aspect, the present invention relates to the aqueous gelatin composition of the fifth aspect of the present invention for use in dispensing devices, preferably 3D printing devices.

In a seventh aspect, the present invention relates to a construct which comprises the gelatin of the present invention, or the gelatin composition of the present invention or the aqueous gelatin composition of the present invention, and wherein the gelatin is gelled. Preferably said construct is produced by dispensing, preferably by 3D printing.

In an eight aspect, the present invention relates to a process for making a construct, characterized in that it comprises the steps of
a. providing an aqueous gelatin composition, and
b. feeding the aqueous gelatin composition in a dispensing device, preferably a 3D printer, and
c. operating the dispensing device such as to provide a construct, characterized in that the aqueous gelatin composition is according to the present invention.

In a ninth aspect, the present invention relates to the use of the gelatin of the present invention or of the gelatin composition of the present invention, or of the aqueous gelatin composition of the present invention to increase the dispensing time window in dispensing systems, preferably to increase the printability time window in 3D printing systems. In other words, the present invention relates to the use of the gelatin of the present invention or of the gelatin composition of the present invention, or of the aqueous gelatin composition of the present invention to obtain a dispensing time window in dispensing devices as defined above, preferably a printing time window in 3D printing devices, of from above 15 minutes to 3 hours, more preferably from 20 minutes to 2 hours and 30 minutes, even more preferably from 30 minutes to 2 hours, yet even more preferably from 40 minutes to 2 hours, yet even more preferably from 40 minutes to 2 hours, yet even more preferably from 50 minutes to 2 hours, yet even more preferably from 1 hour to 2 hours.

In a tenth aspect, the present invention relates to the use of the gelatin of the present invention, or of the gelatin composition of the present invention or of the aqueous gelatin composition of the present invention to reduce or delay the clogging of dispensing systems during dispensing process, preferably to reduce or delay the clogging of 3D printing systems during 3D printing process.

In an eleventh aspect, the present invention relates to the use of the gelatin of the present invention, or of the gelatin composition of the present invention or of the aqueous gelatin composition of the present invention in the production of scaffolds, engineered tissues, devices or micro-devices suitable for therapeutic purposes, diagnostic purposes, research purposes and the like, said production being done by dispensing, preferably by 3D printing.

In a twelfth aspect, the present invention relates to the use of the construct of the present invention as a scaffold for tissue engineering, for regenerative medicine, for diagnostic purposes, for drug testing and the like.

### DETAILED DESCRIPTION

### Definitions

As used in the present description, "gelatin" refers to its well-known definition in the art, i.e. it is a linear polymer resulting from partial hydrolysis of collagen. Said partial hydrolysis may be obtained by chemical, enzymatic and/or heat treatment of collagen. In the case of gelatin, hydrolysis of collagen is said to be partial because gelatin keeps some functional properties (such as the ability to form gels) contrary to collagen hydrolysate, which have lost such functional properties of forming gels. Gelatin may be derived from collagen sourced of any suitable type of connective animal tissue, particularly from bones, teeth and hides, preferably collagen is sourced from bones and/or hides. Collagen may be derived from different animals such as pork, beef, sheep, goat, horse, deer, chicken, fish and the like. Preferably, however, collagen and thus also gelatin, is derived from pork and/or beef. Standard gelatin requires to be heated in order to dissolve in water. Upon cooling standard gelatin forms a gel, the strength of said gel depends on the specific properties of the gelatin such as molecular weight distribution and molecular conformation. Gelatin is mostly characterized by its gel strength, expressed in g Bloom. Further, gelatin is also characterized by a certain viscosity, expressed in mPa.s. Among others, further properties of gelatin are bulk weight or bulk density and particle size.

As used in the present description, "dispensing systems" or "dispensing devices" refer to apparatus able to deposit a composition, typically as a liquid or paste, onto a surface in the form of dots, lines and any other suitable shapes. Simply stated, said composition is pushed from a reservoir onto a surface through a head, which is typically a nozzle. The head may be driven by pressure, piezoelectric motor or laser-assisted jetting. Depending on the apparatus and on the composition, the head may be of different sizes and different openings, also the need for more or less pressure will depend on the apparatus and composition to be dispensed. Dispensing may be done by extrusion, spraying, jetting and the like. Casting where higher shear stress is used, which is a process comprising filling a mold with a composition to create a specific shape is also understood to be a dispensing process. Casting is however not the preferred way of dispensing. Dispensing devices are preferably computer-controlled systems. A suitable software allows the user to enter a pre-determined shape which is then dispensed on a surface by the dispensing device to provide a construct. Thus a dispensing device may be a 3D printer, an ink jet printer, a single jet dispensing device, and the like. Preferably, the dispensing device is a 3D printing system, i.e. a 3D printer. Preferably, in the present description, dispensing comprises 3D printing.

As used in the present description, "3D printing" refers to a specific dispensing system. 3D printing systems allow to produce tridimensional shaped products, typically by depositing a composition layer by layer until the desired shape is obtained. 3D printing is, as many other dispensing systems, a computer-controlled system. Preferably in the present invention, 3D printing systems do not require temperature control during printing.

As used in the present description, "dispensing time window" refers to the time during which the composition to be dispensed can be dispensed without encountering dispensing problems due to, for example, the change in rheology of the composition within the reservoir and/or through the dispensing head of the dispensing system. The dispensing time window starts when dispensing begins and ends when the dispensing head gets clogged by the composition or when the dispensing does no longer happen in a suitable, uniform manner, e.g. undesired lumps are formed, dispensing spots are missed and the like, which is disturbing the desired final shape of the construct.

As used in the present description, "crosslinking" refers to the formation of a stable network structure, in particular stable network gel structure, via covalent bonding. Typically, crosslinking is obtained by the action of an external factor (crosslinking factor) such as exposure to heat, exposure to UV light (photo-initiated chain-growth polymerization or crosslinking) and the like, on functionalized molecules. Thus molecules are first functionalized such that crosslinking can happen upon subjection to the right conditions. During functionalization, functional groups are added to the chemical structure of (some of) the molecules by a chemical reaction (chemical functionalization) or by a biochemical reaction (biochemical functionalization) such as an enzymatic reaction for example (enzymatic functionalization). Chemical functionalization may be for example an acrylation reaction, i.e. introducing acrylate substitution groups; a methacrylation reaction, i.e. introducing methacrylate substitution groups, diels -alder, azide-alkyne cycloaddition reaction, thiol-ene chemistry (click chemistry) or a succinylation reaction. Other chemical or biochemical functionalizations are not excluded. Molecules may also be previously treated to add reactive groups in their chemical structure in order to give access to new functional chemical groups which will facilitate crosslinking. Alternatively, crosslinking may also be obtained for example by the reaction of certain components present in a composition with specific reactants, for example, alginate present in a composition will react and crosslink when exposed to calcium ions, for example when the composition is put in a bath comprising calcium ions. For a composition to be crosslinked, it is sufficient that at least one of its components is crosslinked. For example, a composition comprising gelatin and alginate may be crosslinked when it is exposed to calcium ions, in that case only the alginate is in fact crosslinked, however it may be said that the composition is crosslinked.

As used in the present description, "crosslinkable" refers to the ability of a composition to react to an external crosslinking factor causing at least two components of the composition to be crosslinked. A composition can be made crosslinkable by adding functional groups to the chemical structure of (some of) the component(s) of the composition by a chemical or biochemical reaction (see above: functionalization). Alternatively, a composition can be made crosslinkable by adding crosslinking agents (or crosslinking inducers) such as formaldehyde, glutaraldehyde, 1-ethyl-3-(3-dimethylaminopropyl), carbodiimide/N-hydroxysuccinimide, 1,4-Butanediol Diglycidyl Ether, epoxy compounds and the like. Natural crosslinking agents are for example enzymes (for example transglutaminase) and genipin (an agent extracted from gardenia fruit). Such natural crosslinking agents can provide lower cytotoxicity levels. Crosslinking agents like e.g. genipin containing more than one reactive group may react with reactive groups on more than one protein molecule and chemically crosslink the gelatin molecules; the crosslink inducers then become part of the crosslinked structure chemically. Enzymes, e.g. transglutaminases or tyrosinases catalyse the chemical reaction between reactive groups on the protein molecule to form chemical bonds and inter- and intramolecular crosslinks; the enzyme does not become part of the crosslinked network chemically.

The invention will further be illustrated with different embodiments.

In a first aspect, the present invention relates to a gelatin characterized in that
a. it is soluble in an aqueous medium and
b. the first derivative of the gelatin's FTIR-ATR spectrum remains below zero within the 1440-1355 cm⁻¹ spectral region.

The gelatin comprises from 95 weight% (wt%) to 100wt%, preferably at least 96wt%, more preferably at least 97wt%, yet even more preferably at least 98wt%, yet even more preferably at least 99wt% of gelatin on dry basis of gelatin (wt% db). The gelatin may indeed comprise some minor amounts of ashes and/or salts which typically result from the processing of collagen into gelatin.

The gelatin of the present invention is soluble in an aqueous medium. In this context, solubility in an aqueous medium means that when 10g of the gelatin is stirred in 100ml of demi-water at 60°C during 1hour, a clear solution is obtained with no particles visible to the naked eye.

FTIR is a well-known method to characterize the structure of organic components. The FTIR-ATR spectrum of a gelatin sample is obtained with a resolution of 4 cm⁻¹, accumulating 16 scans per spectrum. Spectral measurements are performed within the mid-infrared range of 4500-600 cm⁻¹. Measurement is performed with a diamond triple-bounce ATR accessory (Bruker, Tensor 27). Background air spectrum is collected before each sample measurement. A sample is directly compressed on attenuated total reflectance (ATR) crystal prior to analysis without any preliminary preparation. After measurement of a gelatin sample, ethanol and distilled water are used to clean the diamond ATR crystal, which has to dry before performing a next measurement. Between each sample measurement a blank measurement is done (i.e. measurement without presence of sample). The spectra is depicted in transmittance mode.

OriginPro8.5 is used to calculate the first derivative of the spectra within the 1440 - 1355 cm-1 wavenumber range. The signal is smoothed by applying a quadratic Savitzky-Golay filter with 5-points.

The gelatin of the present invention may be obtained by a process of flash hydrolysis followed by rapid evaporation of water.

Preferably the gelatin is further characterized in that it shows a shift of the peak in the region of the Amide I band (C=O stretch) (1660-1600 cm⁻¹) of about 1 cm⁻¹, preferably of about 0.5 cm⁻¹ towards the high ranges of wavenumber, compared to the gelatin starting material.

More preferably, the gelatin is further characterized in that it shows a shift of the peak in the region of the C-N-H bending band (1565-1500 cm⁻¹) of about 9 cm⁻¹, preferably of about 7 cm⁻¹ , more preferably about 5 cm⁻¹ towards the lower ranges of wavenumber, compared to the gelatin starting material.

Preferably, the gelatin is in powder form, particle size may vary but is preferably 70 to 8 mesh, more preferably 60 to 15 mesh, even more preferably 60 to 20 mesh, yet even more preferably 50 to 30 mesh, yet even more preferably 40 to 30 mesh.

Preferably, the gelatin has a gel strength of above 50g Bloom, preferably from 50g to 360g Bloom, preferably 80g to 350g Bloom, more preferably 100g to 350g Bloom, yet even more preferably 120g to 340g Bloom, yet even more preferably 150g to 340g Bloom, yet even more preferably 180g to 330g Bloom, yet even more preferably 200g to 330g Bloom, yet even more preferably 250g to 330g Bloom, yet even more preferably 270g to 320g Bloom, yet even more preferably 280g to 320g Bloom.

In one embodiment, the gelatin of the present invention is substantially not functionalized. Substantially not functionalized means that the degree of functionalization, in particular the degree of chemical functionalization is less than 5%, more preferably from 3% to 0%, yet even more preferably from 1% to 0%, most more preferably from 0.1% to 0%. Functionalization is as defined above.

In another embodiment, the gelatin of the present invention is functionalized, meaning that the gelatin of the present invention is subjected to a functionalization reaction. Functionalization is as defined above. It is an advantage of the present gelatin that due to its high amounts of gelatin, a functionalization reaction is easier to perform, compared to other dispensing material where the composition renders a functionalization reaction more difficult to perform, or makes it more difficult to find the right reaction conditions. Preferably the degree of functionalization is from 30% to 95%, more preferably from 35 to 90%, yet even more preferably from 40 to 85%, yet even more preferably from 45 to 80%, yet even more preferably from 50 to 75%, yet even more preferably from 55 to 70%, yet even more preferably from 60 to 65%. The levels of functionalization will depend on the application. Higher degrees of functionalization is indicated for applications that require a more stable structure in physiological conditions of pH and temperature. Preferably the gelatin is chemically functionalized, more preferably the gelatin is acrylated or methacrylated, more preferably the gelatin is methacrylated.

Thus the present invention further relates to a functionalized gelatin, preferably a methacrylated gelatin characterized in that it is prepared by providing a gelatin according to the present invention and subjecting the gelatin of the present invention to a functionalization reaction, preferably to a methacrylation reaction.

In a further embodiment, the gelatin comprises functionalized gelatin of the present invention and substantially non functionalized gelatin of the present invention. Thus from 1 to 99wt%, preferably from 5 to 90wt%, more preferably from 10 to 80wt%, even more preferably from 20 to 60wt%, yet even more preferably from 30 to 50wt% yet even more preferably from 40 to 50wt% of the gelatin is functionalized and from 1 to 99wt%, preferably from 5 to 90wt%, more preferably from 10 to 80wt%, even more preferably from 20 to 60wt%, yet even more preferably from 30 to 50wt% yet even more preferably from 40 to 50wt% is substantially not functionalized. The degree of functionalization is as described here above.

During methacrylation, methacrylate groups are introduced on the gelatin molecules through a well-known process: typically gelatin is dissolved in a solvent, typically phosphate buffer saline (PBS) or dimethyl sulfoxide solvent (DMSO), methacrylic anhydride is then directly added to said gelatin solution, the methacrylation reaction is conducted at about 50°C. The degree of methacylation can be controlled by the amount of methacrylic anhydride added. The gelatin of the present invention may be methacrylated and may have a degree of methacrylation as mentioned above.

During acrylation, acrylate groups are introduced on the gelatin molecules through a well-known process. Reference is made for example to Billiet, I.T. (2014) "Gelatin Functionalizations for Cell Embedding in 3D Geometries Using Rapid Prototyping Technology".

Preferably further, the gelatin of the present invention comprises less than 100 Endotoxin Units (EU), more preferably less than 80 EU, even more preferably less than 60 EU, yet even more preferably less than 40 EU, yet even more preferably less than 30 EU, yet even more preferably less than 10 EU per gram of gelatin, expressed on as is basis. Endotoxins may be removed by clearance filters for endotoxins removal such as charged filters. Such charged filters are available and can be for example Mustang E^{®} membrane and Mustang Q^{®} membrane (from Pall), Sartobind^{®} filter (from Sartorious). The LAL test (acronym for Limulus Amebocyte Lysate) is a test for the determination of bacterial endotoxins, which uses an Amebocyte Lysate of the Limulus crab.

Further preferably the gelatin of the present invention is purified by using one or more of the following techniques: ultrafiltration, diafiltration, filtration with depth filters. These filtration techniques are useful to (partially) remove for example undesired toxins, bacteria, product fractions and the like.

Preferably the gelatin of the present invention comprises 5wt% or less than 5wt%, more preferably 4wt% or less than 4wt%, even more preferably 3wt% or less than 3wt%, yet even more preferably 2wt% or less than 2wt%, yet even more preferably 1wt% or less than 1wt%, yet even more preferably 0.5wt% or less than 0.5wt%, yet even more preferably 0.1wt% or less than 0.1wt% of additives, on dry basis of the gelatin. More preferably, the gelatin of the present invention comprises substantially no additives. Additives may be rheological enhancers, flowability enhancers and the like, such as for example glycols, starches, alginates and the like. It is an advantage of the present gelatin to comprises such low amounts of additives in the ranges mentioned above, in particularly to comprise substantially no additives, in particular when the gelatin is used to produce scaffolds for use as ECM with living cells as the additives have a negative effect on cell survival rates. Many current material for producing scaffolds for use as ECM nowadays comprise additives in order to allow the materials to be dispensed, in particular to be 3D printed into said scaffolds.

The gelatin of the present invention is alternatively characterized in that when dispensed
a. as a 10wt% solution in water, and
b. under pressure from 10 to 80kPa, preferably from 20 to 60kPa and
c. at room temperature, and
d. through a nozzle of from 0.01 to 3mm,
   said solution keeps a substantially consistent flow behavior for at least 15 minutes. Dispensing is to be done at most 3hours after preparation of said solution. The solution is prepared by dissolving gelatin at 50°C for 30minutes after which the solution may be returned to room temperature (18 to 25°C, preferably 20 to 25°C).

Preferably, dispensing is done at around the gelation temperature of the 10% solution of gelatin, i.e. at a temperature in the range of about 10°C less to 10°C more than the gelation temperature, preferably 5°C less to 5°C more than the gelation temperature. The possibility to print at said temperature is an advantage of the present invention, indeed, existing gelatin-based printing material cannot be printed at a temperature around the gelation temperature unless specific measures are taken such as for example the addition of specific additives. Also as mentioned above, existing gelatin-based printing material are often printed in heated system, more than at least 10°C, at least 15°C or even at least 20°C above the gelation temperature.

Advantageously also, at equal concentration, dispensing can be done at lower pressure with the gelatin of the present invention, compared to existing gelatin-based printing material.

Preferably, said solution keeps a substantially consistent flow behavior for a time of from above 15 minutes to 3 hours, more preferably from 20 minutes to 2 hours and 30 minutes, even more preferably from 30 minutes to 2 hours, yet even more preferably from 40 minutes to 2 hours, yet even more preferably from 40 minutes to 2 hours, yet even more preferably from 50 minutes to 2 hours, yet even more preferably from 1 hour to 2 hours.

In a second aspect, the present invention relates to the gelatin of the first aspect of the present invention for use in dispensing devices, preferably for use in a 3D printer. It has been found that said gelatin is particularly suitable for dispensing, in particular for 3D printing, such as for use as a bio-ink in 3D printing. The present invention also relates to the gelatin of the first aspect of the present invention for use in the construction of scaffold for medical applications, preferably for tissue engineering, for regenerative medicine, for implants, for medical devices, for drug discovery and drug testing; or for cosmetic applications, preferably a scaffold for testing of cosmetic products; and the like. The gelatin can be dispensed, without the need of having a temperature controlled dispensing system and without the need of having other ingredients added, or additives added or other solutions that are typically used in the art to be able to dispense gelatin. Preferably dispensing is done under pressure, more preferably at room temperature. Dispensing, and particularly printing, is done by putting the material into an aqueous solution. The solvent may be a phosphate buffer, bovine serum albumin (BSA), water, preferably the solvent is a phosphate buffer or BSA, more preferably a phosphate buffer. Advantageously, the gelatin is put in solution at a temperature of from 30 to 60°C, more preferably from 40 to 55°C, yet even more preferably from 40 to 50°C and allowed to cool down to room temperature before dispensing. It is preferable to start the dispensing process before a period of maximum 3 hours after cool down.

Further advantage of the present gelatin is that it can be dispensed as a highly concentrated aqueous solution, i.e. aqueous solution containing up to 30wt% of the gelatin of the present invention, preferably the gelatin of the present invention is used in dispensing devices as a solution comprising from 1 to 30wt%, more preferably from 5 to 25wt%, even more preferably from 7.5 to 20wt%, yet even more preferably from 7.5 to 15wt%, yet even more preferably from 10 to 15wt% of the gelatin of the present invention.

In a third aspect, the present invention relates to a gelatin composition comprising the gelatin of the first aspect of the present invention and further ingredients.

Preferably the gelatin composition is in powder form.

The gelatin composition comprises one or more further ingredients chosen from bulking agents, hydrocolloids, proteins, anticaking agents and the like. Preferably the gelatin composition of the present invention comprises from 40wt% to 95wt%, more preferably from 45wt% to 95wt%, even more preferably from 50wt% to 95wt%, yet even more preferably from 55wt% to 95wt%, yet even more preferably from 60wt% to 95wt%, yet even more preferably from 65wt% to 95wt%, yet even more preferably from 70wt% to 95wt%, yet even more preferably from 75wt% to 95wt%, yet even more preferably from 80wt% to 95wt%, yet even more preferably from 85wt% to 95wt% of the gelatin of the present invention, on dry basis of the gelatin composition. Thus preferably the gelatin composition of the present invention comprises from 5wt% to 60wt%, more preferably from 5wt% to 55wt%, even more preferably from 5wt% to 50wt%, yet even more preferably from 5wt% to 45wt%, yet even more preferably from 5wt% to 40wt%, yet even more preferably from 5wt% to 35wt%, yet even more preferably from 5wt% to 30wt%, yet even more preferably from 5wt% to 25wt%, yet even more preferably from 5wt% to 20wt%, yet even more preferably from 5wt% to 15wt% of the further ingredients as defined herein.

Bulking agents are well-known in the art and refer to material which do not possess any functionality, inert products, such as sugars, mono-, di- and tri-saccharides, maltodextrins, fibers, protein hydrolysates (such as collagen hydrolysates for example) and the like.

Hydrocolloids may be one or more of alginate, pectin, carrageenan, gellan, agar, starch, xanthan gum, locust bean gum, guar gum, gum karaya, gum tragacanth, gum Arabic, cellulose derivatives, chitosan and the like. Preferably the hydrocolloids are alginate or carrageenan, more preferably alginate.

Proteins may be one or more of keratin, elastin, fibroin, thrombin, albumin, and the like. Protein may also be derived from collagen raw materials.

In an embodiment, the gelatin composition comprises functionalized gelatin of the present invention and regular gelatin (i.e. not according to the present invention) which is not functionalized. The gelatin composition preferably comprises from 1 to 99wt%, preferably from 5 to 90wt%, more preferably from 10 to 80wt%, even more preferably from 20 to 60wt%, yet even more preferably from 30 to 50wt% yet even more preferably from 40 to 50wt% of functionalized gelatin of the present invention (the degree of functionalization is as described above), and from 1 to 99wt%, preferably from 5 to 90wt%, more preferably from 10 to 80wt%, even more preferably from 20 to 60wt%, yet even more preferably from 30 to 50wt% yet even more preferably from 40 to 50wt% of regular gelatin, non-functionalized.

Further preferably the gelatin composition of the present invention has been purified by using one or more of the following techniques: ultrafiltration, diafiltration, filtration with depth filters. These filtration techniques are useful to remove or at least substantially lower the amount of bacteria, endotoxins, fats, non-collagen proteins, fibres and the like.

In a fourth aspect the present invention relates to the gelatin composition of the third aspect of the present invention for use in dispensing devices, preferably in 3D printer. It has been found that said gelatin composition is particularly suitable for dispensing, in particular 3D printing, such as for use as a bio-ink in 3D printing. The present invention also relates to the gelatin composition of the third aspect of the present invention for use in the construction of scaffold for medical applications, preferably for tissue engineering, for regenerative medicine, for implants, for drug discovery and drug testing, or for cosmetic applications, preferably a scaffold for testing of cosmetic products and the like, or for food applications, such as for example personalized meals, reconstituted meals and the like.

The gelatin composition can be dispensed when put into solution without the need of having a temperature-controlled dispensing system and without the need of having additives added or other solutions that are typically used in the art to be able to dispense gelatin composition. Preferably dispensing is done under pressure, more preferably at room temperature. Dispensing, and particularly printing, is done with the gelatin composition put into an aqueous solution. The solvent may be a phosphate buffer, BSA, water, preferably it is a phosphate buffer or BSA, more preferably a phosphate buffer. Advantageously, the gelatin composition is put in solution at a temperature of from 30 to 60°C, more preferably from 40 to 55°C, yet even more preferably from 40 to 50°C and allowed to cool down to room temperature before dispensing. It is preferable to start the dispensing process maximum before a period of 3 hours after cool down.

A further advantage of the present gelatin composition is that it can be dispensed as a highly concentrated aqueous solution, i.e. aqueous solution containing up to 30wt% of the gelatin composition, preferably the present gelatin composition is used in dispensing devices as a solution comprising from 1 to 30wt%, more preferably from 5 to 25wt%, even more preferably from 7.5 to 20wt%, yet even more preferably from 7.5 to 15wt%, yet even more preferably from 10 to 15wt% of the gelatin of the present invention.

In a fifth aspect, the present invention relates to an aqueous gelatin composition comprising from 1 to 30wt%, preferably from 5 to 25wt%, even more preferably from 7.5 to 20wt%, yet even more preferably from 7.5 to 15wt%, yet even more preferably from 10 to 15wt% of the gelatin of the first or second aspect of the present invention or of the third or fourth aspect of the present invention and from 70 to 99wt%, preferably from 95 to 75wt%, even more preferably from 80 to 92.5wt%, yet even more preferably from 85 to 92.5wt%, yet even more preferably from 85 to 90wt% of an aqueous medium.

The aqueous medium may be a phosphate buffer, bovine serum albumin (BSA), water, preferably the solvent is a phosphate buffer or BSA, more preferably a phosphate buffer.

Preferably, the aqueous gelatin composition of the present invention comprises various components, preferably cell growth components, i.e. components which have a beneficial effect on the growth of living plant, human or animal cells. Such components may be cytokines (Adiponectin Human) cell growth promoters or growth factors, such as (EGF, FGF, NGF, PDGF, VEGF, IGF, GMCSF, GCSF, TGF, Erythropieitn, TPO, BMP, HGF, GDF, Neurotrophins, MSF, SGF, GDF); nutrients, such as glucose, yeast extract; salts, such as potassium chloride, sodium chloride and the like; proteins, such as thrombin transferrin, albumin, peptones and the like.

Preferably, the aqueous gelatin composition of the present invention further comprises living organisms, preferably human, animal and/or plant cells. Said living organisms may be encapsulated or non-encapsulated.

Advantageously, the aqueous gelatin composition of the present invention is prepared by bringing all components in solution at a temperature of from 30 to 60°C, preferably from 40 to 55°C, yet even more preferably from 40 to 50°C and letting the aqueous composition cool down to room temperature before further use.

Further preferably the aqueous gelatin composition of the present invention has been purified by using one or more of the following techniques: ultrafiltration, diafiltration, filtration with depth filters. These filtration techniques are useful to remove or at least substantially lower the amount of bacteria, endotoxins (removal of e.g. from 10 to 90% of endotoxins), fats, non-collagen proteins, fibres and the like.

In a sixth aspect, the present invention relates to the aqueous gelatin composition of the present invention for use in dispensing devices, preferably in 3D printer. It has been found that said aqueous gelatin composition is particularly suitable for dispensing, in particular for use as a bio-ink in 3D printing. The present invention also relates to the aqueous gelatin composition of the present invention for use in dispensing devises, preferably in 3D printers, in the construction of scaffold for medical applications, preferably for tissue engineering, for regenerative medicine, for implants, for drug discovery and drug testing, or for cosmetic applications, preferably a scaffold for testing of cosmetic products, or food applications and the like. Also the present aqueous gelatin composition is very suitable for dispensing, preferably 3D printing.

The aqueous gelatin composition can be dispensed without the need of having a temperature-controlled dispensing system and without the need of having additives added or other solutions that are typically used in the art to be able to dispense aqueous gelatin compositions. Preferably dispensing is done under pressure, more preferably at room temperature.

Advantageously, the aqueous gelatin composition is heated to a temperature of from 30 to 60°C, more preferably from 40 to 55°C, yet even more preferably from 40 to 50°C and allowed to cool down before dispensing. It is not desired to keep the cooled aqueous gelatin composition for a period of time of more than 3hours at room temperature before starting the dispensing process.

In a seventh aspect, the present invention relates to a construct, comprising the gelatin of the present invention, the gelatin composition of the present invention and/or the aqueous gelatin composition of the present invention, wherein the gelatin is gelled. Preferably said construct being produced by dispensing, more preferably by 3D printing.

As mentioned above, the gelatin of the present invention may or may not be functionalized. When functionalized, the construct is crosslinkable, and crosslinking may be done by application of the right crosslinking conditions (such as applying a photoinitiator for example). When crosslinking reaction has been performed, it may be said that the construct is crosslinked. Crosslinked constructs, especially comprising gelatin which is a natural ingredient that melts at body temperature, are very stable and can be introduced in the body or used at temperatures above the melting temperature of gelatin, without the fear that the construct would melt away or show deformations.

Preferably crosslinking takes place immediately after dispensing (filament deposition) to ensure self-supporting properties of the 3D-construct, to avoid collapsing of the 3D construct after printing.

The construct may further comprise one or more of other components such as carbohydrates, salts, living cells, cell growth promoters and enzymes.

The construct may comprise the gelatin, the gelatin composition and/or the aqueous gelatin composition of the present invention ('gelatin comprising compositions') and other further components due to for example dispensing the construct with multiple headed nozzles, where one or more nozzles dispensing the gelatin comprising composition and the other one or more nozzles dispensing another type of suitable composition which can be dispensed and is preferably biocompatible, such as a composition comprising hydrocolloids, for example, alginate; proteins; carbohydrates and the like.

In an eight aspect, the present invention relates to a process for making a construct, characterized in that the process comprises the steps of
a. providing an aqueous gelatin composition, and
b. feeding the aqueous gelatin composition in a dispensing device, preferably a 3D printer, and
c. operating the dispensing device such as to provide a construct,
   characterized in that the aqueous gelatin composition is according to the present invention.

The construct is as defined herein.

Operating the dispensing device may be done at a controlled temperature such as at a temperature of from above 25 to 40°C, however advantageously with the present aqueous gelatin composition, dispensing is done without controlled temperature system, at room temperature (18 to 25°C, preferably 20 to 25°C). Preferably, dispensing is is done at around the gelation temperature of the 10% solution of gelatin, i.e. at a temperature in the range of about 10°C less to 10°C more than the gelation temperature, preferably 5°C less to 5°C more than the gelation temperature. The possibility to print at said temperature is an advantage of the present invention, indeed, existing gelatin-based printing material cannot be printed at a temperature around the gelation temperature unless specific measures are taken such as for example the addition of specific additives. Also as mentioned above, existing gelatin-based printing material are often printed in heated system, more than at least 10°C, at least 15°C or even at least 20°C above the gelation temperature.

Preferably the construct is made by dispensing, preferably by printing, layer above layer or dot above dot of the aqueous gelatin composition. If needed the construct may also be a single layer or dot of the aqueous gelatin composition. In fact, any suitable construct may be printed, in accordance with the possibilities of the used dispensing system, preferably of the used 3D printer software.

Once the construct is made, it may be crosslinked, provided either that the aqueous gelatin composition was functionalized to be crosslinked or provided that enzymes have been added which can cause crosslinking. Preferably crosslinking takes place right after dispensing (filament deposition), i.e. the moment of cross-linking depending on the viscoelastic properties of the dispended material, to ensure self-supporting properties of the 3D-construct, thus cross-linking is done on time after dispensing to avoid collapsing of the 3D construct after dispensing or printing.

It is also possible to start the crosslinking reaction whilst dispensing, either directly from the start of the dispensing step or at any suitable time after the start of the dispensing step, before the end of the dispensing step.

Typically, accurate time control during the dispensing process is important when crosslinking agents are present in the aqueous gelatin composition because the crosslinking reaction may cause detrimental viscosity increase inside the nozzle while dispensing. In this case, thermostability of the gelatin of the present invention is advantageous as it allows to prevent the combined effect of physical gel formation together with increments of viscosity caused by (chemical) crosslinking, making it possible to have a more flexible time control during dispensing process.

The present invention further relates to the use of the gelatin of the present invention, the gelatin composition of the present invention or the aqueous gelatin composition of the present invention to increase the dispensing time window in dispensing systems, preferably to increase the printability time window in 3D printing systems.

This effect is due to the fact that said gelatin, gelatin composition and aqueous gelatin compositions show little to no viscosity increase during printing, also at room temperature, in fact even at around the gelation temperature of the gelatin, as defined above , for a period of time of up to 3 hours, preferably of above 15 minutes to 3 hours, more preferably from 20 minutes to 2 hours and 30 minutes, even more preferably from 30 minutes to 2 hours, yet even more preferably from 40 minutes to 1 hour and 30 minutes, yet even more preferably from 40 minutes to 1 hour. In comparison, previous compositions comprising gelatin typically only allowed a dispensing time window of about 15 minutes or less. As a results, clogging of the dispensing head or nozzle, preferably printing nozzle does not happen until after 3 hours of dispensing, preferably printing, preferably not until after a period of above 15 minutes to 3 hours, more preferably from 20 minutes to 2 hours and 30 minutes, even more preferably from 30 minutes to 2 hours, yet even more preferably from 40 minutes to 2 hours, yet even more preferably from 40 minutes to 2 hours, yet even more preferably from 50 minutes to 2 hours, yet even more preferably from 1 hour to 2 hours.

Further, the present invention relates to the use of the gelatin, gelatin composition and aqueous gelatin compositions of the present invention in the preparation, preferably by dispensing, more preferably by 3D printing, of a scaffold for medical applications, preferably for tissue engineering, for regenerative medicine, for implants, for drug discovery and drug testing, or for cosmetic applications, preferably a scaffold for testing of cosmetic products, and the like. Also, the present invention relates to the use of the gelatin, gelatin composition and aqueous gelatin compositions of the present invention in the preparation of food applications such as customized meals, reconstituted foods, bakery applications and the like.

Further, the present invention relates to the use of the construct of the present invention in the preparation of a scaffold for medical applications, preferably for tissue engineering, for regenerative medicine, for implants, for drug discovery and drug testing, or for cosmetic applications, preferably a scaffold for testing of cosmetic products, and the like.

Further, the present invention relates to a process for identifying a gelatin suitable for use in dispensing device, preferably for use in 3D-printing devices, said process comprises the steps
a. taking a gelatin powder
b. performing FTIR-ATR spectrometry and solubility test in aqueous medium of said gelatin
c. concluding that said gelatin is suitable for use in said dispensing device when the first derivative of the gelatin's FTIR-ATR spectrum remains below zero within the 1440-1355 cm⁻¹ spectral region and the gelatin is soluble in aqueous medium.

### Printability/printing behavior:

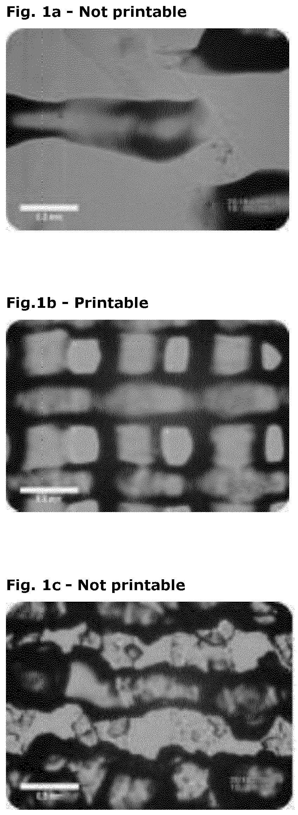

Fig 1 a, b and c exemplify the difference between printability and non-printability. In Fig 1a it can be seen that the composition is not printable as there is no shape fidelity. In Fig 1b it can be seen that the composition is printable, it has a good consistency for extrusion-based 3D printers. In Fig 1c it can be seen that the composition is not printable due to clogging of the nozzle causing disturbance in shape of the construct, the experiment required pausing of the printing job for nozzle replacement.

The present invention will be further illustrated in the non-limiting examples.

### EXAMPLES

### Example 1: production of gelatin according to the present invention

A solution of acid treated porcine gelatin of high bloom (300g) (T=60°C) at a concentration of 35wt% is submitted to flash hydrolysis by heating to achieve a product temperature of 120°C for 60 seconds. The heated gelatin is then submitted to evaporation by roll-coating the heated gelatin solution on the surface of a rotating cylinder heated by steam at 4.5 bar. The adhering thin layer of product on the surface of the cylinder is rapidly dried and scratched off by the action of a sharp knife. The gelatin so produced is soluble in water.

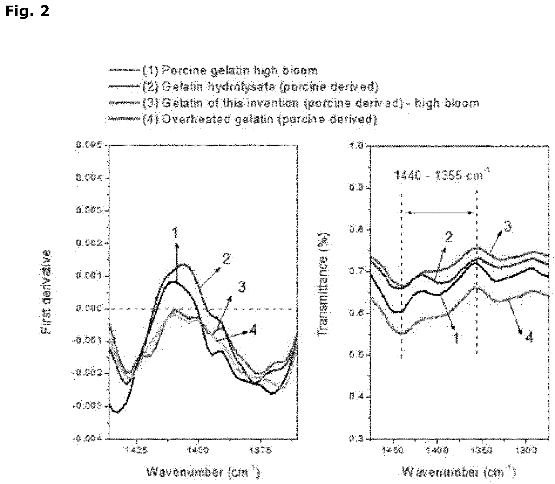

The FTIR-ATR spectrum of the gelatin so produced is measured. The first derivative and the transmittance is depicted in figure 2 (3).

FTIR-ATR spectrum of following material are also depicted in figure 2:
- Regular porcine gelatin of high bloom (300g) (1)
- Porcine derived gelatin hydrolysate (2)
- Overheated porcine derived gelatin (4). Said gelatin has been exposed to 150°C for 2 hours and is not soluble in water.

As depicted in figure 2, the first derivative of the spectrum of the gelatin of the present invention remains below zero within the 1440-1355 cm⁻¹ spectral region. Gelatin (1) and the hydrolysed gelatin (2) depict within that specific spectral region two zero-crossing values, i.e. the derivative crosses zero twice within that region.

### Example 2: preparation of a construct with gelatin according to the invention and with regular gelatin

Comparative experiment demonstrating the printing behavior of gelatin from example 1 in comparison with regular gelatin was conducted. Both gelatins are derived from porcine source and present bloom higher than 280g, about 300g. On a Cellink Inkredible 3D printer, constructs are is prepared with the gelatin according to example 1 and with the regular gelatin.

The gelatin of example 1 and the regular gelatin are each dispersed in a glass vial containing PBS (phosphate buffer solution, pH7.4) to produce a solution at a concentration of 10w/w%. The dispersion of the powder in solution is performed using a vortex mixer (3000 rpm). The vials containing 10mL of solution are then placed in a water bath at 60 °C for 1 hour. While warm, the solutions are then each poured into a 3mL syringe from the 3D printer.

The 2 syringes are placed on the bench and allowed to cool at ambient temperature conditions (about 20°C).

The syringe containing the gelatin according to example 1 is placed in the printhead 1 (PH1) and the syringe containing the regular gelatin is placed in the printhead 2 (PH2). Printing of 6 lattice structures was initiated simultaneously by PH1 and PH2.

The printing settings applied on the Slic3r software for printing of a lattice structure are adjusted as follows:

| Nozzle size | Printing speed | Infill | Pressure PH1 | Pressure PH2 | Temperature |
|---|---|---|---|---|---|
| 25G (0.3 mm) | 150 mm min-1 | 12% | 85-100kPa | 130-140kPa | 23-25 ^{O}C |

The gelatin according to example 1 requires extrusion pressure of 85-100kPa while the regular gelatin requires a pressure of 130-140kPa in order to have filament formation.

| 12 layer count lattice structure | Time required to print (mins) | Gelatin from example 1 | Regular gelatin |
|---|---|---|---|
| Lattice 1 | 19m:15s | Complete | Complete |
| Lattice 2 | 18m:03s | Complete | Complete |
| Lattice 3 | 18m:06s | Complete | Not initiated due to nozzle blockage |
| Lattice 4 | 19m:08s | Complete | Not initiated due to nozzle blockage |
| Lattice 5 | 17m:54s | Complete | Not initiated due to nozzle blockage |
| Lattice 6 | 18m:01s | Complete | Not initiated due to nozzle blockage |

With the regular gelatin, the printing of a third lattice construct is not possible due to complete blockage of the nozzle. With the gelatin according to example 1, it is possible to print up to 6 lattice constructs, printing can be done up to 105 minutes without any blockage of the nozzle and uniform filament production, the flow behavior remains uniform during the entire printing operation.

## Claims

1. A gelatin **characterized in that**
a. it is soluble in aqueous medium and
b. the first derivative of the gelatin's FTIR-ATR spectrum remains below zero within the 1440-1355 cm -1 spectral region

2. The gelatin of claim 1 further **characterized in that** the degree of chemical functionalization is less than 5%, more preferably from 3% to 0%, yet even more preferably from 1% to 0%, most more preferably from 0.1% to 0%..

3. The gelatin according to claim 1, further **characterized in that** it is functionalized, preferably chemically functionalized.

4. The functionalized gelatin according to claim 3, wherein the gelatin is acrylated or methacrylated, more preferably the gelatin is methacrylated..

5. The gelatin of any one of the previous claims further **characterized in that** it comprises 5wt% or less than 5wt% additives, preferably wherein the additives are selected from rheological enhancers and/or flowability enhancers.

6. The gelatin of any one of the previous claims further **characterized in that** it comprises less than 100 Endotoxin Units (EU) per gram of gelatin composition.

7. Use of the gelatin of any one of claims 1 to 6 in dispensing devices, preferably in a 3D printing system, preferably wherein dispensing is done under pressure, preferably further at room temperature.

8. Use of the gelatin of according to any one of claims 1 to 7, in the construction of a scaffold for medical applications, preferably for tissue engineering, for regenerative medicine, for implants, for drug discovery and drug testing, or for cosmetic applications, preferably a scaffold for testing of cosmetic products, wherein construction is done by dispensing, preferably by 3D printing.

9. A gelatin composition comprising the gelatin of any one of claims 1 to 8 and one or more further ingredient, chosen from the group comprising bulking agents, hydrocolloids and proteins.

10. The gelatin composition of claim 9 further **characterized in that** it comprises from 55 to 90wt% of the gelatin and from 10 to 45wt% of the one or more further ingredient.

11. An aqueous gelatin composition comprising from 1 to 30wt% of the gelatin of any one of claims 1 to 8 or the gelatin composition of claims 9 or 10 and from 70 to 99wt% of an aqueous medium, preferably a phosphate buffer or bovine serum albumin, more preferably a phosphate buffer.

12. The aqueous gelatin composition of claim 11 further **characterized in that** it comprises encapsulated or non-encapsulated living organisms, preferably living human, animal and/or plant cells, and/or further **characterized in that** it comprises cell growth components, preferably cell growth promoters, nutrients, salts and/or proteins.

13. Use of the aqueous gelatin composition of any one of claims 11 to 12 in dispensing devices, preferably in 3D printing system, preferably wherein dispensing is done under pressure, preferably further at room temperature.

14. Use of the aqueous gelatin composition of any one of claims 11 to 13 in the construction of a scaffold for medical applications, preferably for tissue engineering, for regenerative medicine, for implants, for drug discovery and drug testing, or for cosmetic applications, preferably a scaffold for testing of cosmetic products.

15. A construct comprising the gelatin according to any one of claims 1 to 8 or the gelatin composition according to any one of claims 9 to 10 characterized the gelatin is gelled, preferably further **characterized in that** the construct is cross-linked, preferably further **characterized in that** the construct is a 3D printed construct.

16. A process for making a construct, **characterized in that** it comprises the steps of
a. providing an aqueous gelatin composition, and
b. feeding the aqueous gelatin composition of step a. in a dispensing device, preferably a 3D printer, and
c. operating the dispensing device such as to provide a construct, **characterized in that** the aqueous gelatin composition is according to any one of claims 11 to 14.

## Patentansprüche

1. Gelatine, **dadurch gekennzeichnet, dass**
a. sie in wässrigem Medium löslich ist und
b. die erste Ableitung des FTIR-ATR-Spektrums der Gelatine innerhalb des Spektralbereichs 1440-1355 cm⁻¹ unter Null bleibt.

2. Gelatine nach Anspruch 1, **dadurch gekennzeichnet, dass** der Grad der chemischen Funktionalisierung weniger als 5 %, stärker bevorzugt 3 % bis 0 %, noch stärker bevorzugt 1 % bis 0 % und am stärksten bevorzugt 0,1 % bis 0 % beträgt.

3. Gelatine nach Anspruch 1, ferner **dadurch gekennzeichnet, dass** sie funktionalisiert, bevorzugt chemisch funktionalisiert ist.

4. Funktionalisierte Gelatine nach Anspruch 3, wobei die Gelatine acryliert oder methacryliert ist, stärker bevorzugt die Gelatine methacryliert ist.

5. Gelatine nach einem der vorhergehenden Ansprüche, **dadurch gekennzeichnet, dass** sie 5 Gew.-% oder weniger als 5 Gew.-% Additive enthält, wobei die Additive bevorzugt aus rheologischen Verstärkern und/oder Fließfähigkeitsverstärkern ausgewählt sind.

6. Gelatine nach einem der vorhergehenden Ansprüche, ferner **dadurch gekennzeichnet, dass** sie weniger als 100 Endotoxineinheiten (EU) pro Gramm der Gelatinezusammensetzung umfasst.

7. Verwendung der Gelatine nach einem der Ansprüche 1 bis 6 in Abgabevorrichtungen, bevorzugt in einem 3D-Drucksystem, wobei bevorzugt die Abgabe unter Druck und bevorzugt ferner bei Raumtemperatur erfolgt.

8. Verwendung der Gelatine nach einem der Ansprüche 1 bis 7, bei der Konstruktion eines Gerüsts für medizinische Anwendungen, bevorzugt für Tissue Engineering, für regenerative Medizin, für Implantate, für die Arzneimittelforschung und Arzneimittelprüfung oder für kosmetische Anwendungen, bevorzugt eines Gerüsts zum Prüfen von kosmetischen Produkten, wobei die Konstruktion durch Abgabe, bevorzugt durch 3D-Druck, erfolgt.

9. Gelatinezusammensetzung, umfassend die Gelatine nach einem der Ansprüche 1 bis 8 und einen oder mehrere weitere Inhaltsstoffe, ausgewählt aus der Gruppe, die Füllstoffe, Hydrokolloide und Proteine umfasst.

10. Gelatinezusammensetzung nach Anspruch 9, ferner **dadurch gekennzeichnet, dass** sie 55 bis 90 Gew.-% der Gelatine und 10 bis 45 Gew.-% des einen oder der mehreren weiteren Inhaltsstoffe umfasst.

11. Wässrige Gelatinezusammensetzung, umfassend 1 bis 30 Gew.-% der Gelatine nach einem der Ansprüche 1 bis 8 oder der Gelatinezusammensetzung nach Anspruch 9 oder 10, und 70 bis 99 Gew.-% eines wässrigen Mediums, bevorzugt eines Phosphatpuffers oder Rinderserumalbumins, stärker bevorzugt eines Phosphatpuffers.

12. Wässrige Gelatinezusammensetzung nach Anspruch 11, ferner **dadurch gekennzeichnet, dass** sie eingekapselte oder nicht eingekapselte lebende Organismen, bevorzugt lebende menschliche, tierische und/oder pflanzliche Zellen umfasst, und/oder ferner **dadurch gekennzeichnet, dass** sie Zellwachstumskomponenten, bevorzugt Zellwachstumsförderer, Nährstoffe, Salze und/oder Proteine, umfasst.

13. Verwendung der wässrigen Gelatinezusammensetzung nach einem der Ansprüche 11 bis 12 in Abgabevorrichtungen, bevorzugt in 3D-Drucksystemen, wobei die Abgabe bevorzugt unter Druck, bevorzugt bei Raumtemperatur erfolgt.

14. Verwendung der wässrigen Gelatinezusammensetzung nach einem der Ansprüche 11 bis 13 bei der Konstruktion eines Gerüsts für medizinische Anwendungen, bevorzugt für das Tissue Engineering, für die regenerative Medizin, für Implantate, für die Arzneimittelforschung und Arzneimittelprüfung oder für kosmetische Anwendungen, bevorzugt eines Gerüst zum Prüfen von kosmetischen Produkten.

15. Konstrukt, umfassend die Gelatine nach einem der Ansprüche 1 bis 8 oder die Gelatinezusammensetzung nach einem der Ansprüche 9 bis 10, **dadurch gekennzeichnet, dass** die Gelatine geliert ist, bevorzugt ferner **dadurch gekennzeichnet, dass** das Konstrukt vernetzt ist, bevorzugt ferner **dadurch gekennzeichnet, dass** das Konstrukt ein 3D-gedrucktes Konstrukt ist.

16. Verfahren zum Herstellen eines Konstrukts, **dadurch gekennzeichnet, dass** es die Schritte umfasst:
a. Bereitstellen einer wässrigen Gelatinezusammensetzung, und
b. Zuführen der wässrigen Gelatinezusammensetzung aus Schritt a. in eine Abgabevorrichtung, bevorzugt einen 3D-Drucker, und
c. Betreiben der Abgabevorrichtung, um ein Konstrukt bereitzustellen, **dadurch gekennzeichnet, dass** die wässrige Gelatinezusammensetzung eine nach einem der Ansprüche 11 bis 14 ist.

## Revendications

1. Gélatine **caractérisée en ce que** :
a. elle est soluble dans un milieu aqueux et
b. la première dérivée du spectre FTIR-ATR de la gélatine reste inférieure à zéro dans la région spectrale 1 440-1 355 cm⁻¹.

2. Gélatine selon la revendication 1, **caractérisée en outre en ce que** le degré de fonctionnalisation chimique est inférieur à 5 %, de manière davantage préférée de 3 % à 0 %, de manière encore davantage préférée de 1 % à 0 %, et de manière préférée entre toutes de 0,1 % à 0 %.

3. Gélatine selon la revendication 1, **caractérisée en outre en ce qu'**elle est fonctionnalisée, de préférence fonctionnalisée chimiquement.

4. Gélatine fonctionnalisée selon la revendication 3, la gélatine étant acrylée ou méthacrylée, de manière davantage préférée la gélatine étant méthacrylée.

5. Gélatine selon l'une quelconque des revendications précédentes, **caractérisée en outre en ce qu'**elle comprend 5 % en poids ou moins de 5 % en poids d'additifs, de préférence les additifs étant choisis parmi des agents améliorant la rhéologie et/ou des agents améliorant la fluidité.

6. Gélatine selon l'une quelconque des revendications précédentes, **caractérisée en outre en ce qu'**elle comprend moins de 100 unités d'endotoxine (UE) par gramme de composition de gélatine.

7. Utilisation de la gélatine selon l'une quelconque des revendications 1 à 6 dans des dispositifs de distribution, de préférence dans un système d'impression 3D, de préférence la distribution étant effectuée sous pression, de préférence en outre à température ambiante.

8. Utilisation de la gélatine selon l'une quelconque des revendications 1 à 7, dans la construction d'un échafaudage pour des applications médicales, de préférence pour l'ingénierie tissulaire, pour la médecine régénérative, pour des implants, pour la découverte de médicaments et le test de médicaments, ou pour des applications cosmétiques, de préférence un échafaudage pour tester des produits cosmétiques, la construction étant effectuée par distribution, de préférence par impression 3D.

9. Composition de gélatine comprenant la gélatine selon l'une quelconque des revendications 1 à 8 et un ou plusieurs ingrédients supplémentaires, choisis dans le groupe comprenant les agents gonflants, les hydrocolloïdes et les protéines.

10. Composition de gélatine selon la revendication 9, **caractérisée en outre en ce qu'**elle comprend de 55 à 90 % en poids de la gélatine et de 10 à 45 % en poids des un ou plusieurs ingrédients supplémentaires.

11. Composition aqueuse de gélatine comprenant de 1 à 30 % en poids de la gélatine selon l'une quelconque des revendications 1 à 8 ou de la composition de gélatine selon la revendication 9 ou 10 et de 70 à 99 % en poids d'un milieu aqueux, de préférence un tampon phosphate ou de l'albumine sérique bovine, de manière davantage préférée un tampon phosphate.

12. Composition aqueuse de gélatine selon la revendication 11, **caractérisée en ce qu'**elle comprend des organismes vivants encapsulés ou non encapsulés, de préférence des cellules vivantes humaines, animales et/ou végétales, et/ou **caractérisée en outre en ce qu'**elle comprend des composants de croissance cellulaire, de préférence des promoteurs de croissance cellulaire, des nutriments, des sels et/ou des protéines.

13. Utilisation de la composition aqueuse de gélatine selon l'une quelconque des revendications 11 à 12 dans des dispositifs de distribution, de préférence dans un système d'impression 3D, de préférence la distribution étant effectuée sous pression, de préférence en outre à température ambiante.

14. Utilisation de la composition aqueuse de gélatine selon l'une quelconque des revendications 11 à 13 dans la construction d'un échafaudage pour des applications médicales, de préférence pour l'ingénierie tissulaire, pour la médecine régénérative, pour des implants, pour la découverte de médicaments et le test de médicaments, ou pour des applications cosmétiques, de préférence un échafaudage pour tester des produits cosmétiques.

15. Construction comprenant la gélatine selon l'une quelconque des revendications 1 à 8 ou la composition de gélatine selon l'une quelconque des revendications 9 à 10, **caractérisée en ce que** la gélatine est gélifiée, de préférence **caractérisée en outre en ce que** la construction est réticulée, de préférence **caractérisée en outre en ce que** la construction est une construction imprimée en 3D.

16. Procédé de fabrication d'une construction, **caractérisé en ce qu'**il comprend les étapes consistant à :
a. fournir une composition aqueuse de gélatine, et
b. introduire la composition aqueuse de gélatine de l'étape a. dans un dispositif de distribution, de préférence une imprimante 3D, et
c. actionner le dispositif de distribution de manière à fournir une construction,
**caractérisé en ce que** la composition aqueuse de gélatine est selon l'une quelconque des revendications 11 à 14.
